# EUROPÄISCHE PATENTSCHRIFT

(11) **EP 3 256 068 B1**
(45) Veröffentlichungstag und Bekanntmachung des Hinweises auf die Patenterteilung: **17.10.2018**
(21) Anmeldenummer: 16701274.9
(22) Anmeldetag: 20.01.2016
(51) Int. Cl.: A61C 13/01, A61C 13/00, A61C 13/10

(54) **ERSTELLEN EINER WACHS- ODER KUNSTSTOFFBASIS ZUR FERTIGUNG EINER DENTALPROTHESE**
CREATION OF A WAX BASE OR PLASTIC BASE FOR A DENTAL PROSTHESIS
CRÉATION D'UNE BASE DE CIRE OU DE MATIÈRE SYNTHÉTIQUE POUR LA PRODUCTION D'UNE PROTHÈSE DENTAIRE

(30) Priorität: 11.02.2015 DE 102015101978
(43) Veröffentlichungstag der Anmeldung: 20.12.2017
(73) Patentinhaber: Kulzer GmbH, 63450 Hanau (DE)
(72) Erfinder: SAVIC, Novica, 63526 Erlensee (DE); RENZ, Karl-Heinz, 63755 Alzenau (DE); GALL, Silke Maren, 63755 Alzenau (DE)
(74) Vertreter: Schultheiss & Sterzel Patentanwälte PartG mbB
(86) Internationale Anmeldenummer: PCT/EP2016/051082
(87) Internationale Veröffentlichungsnummer: WO 2016/128192

(56) Entgegenhaltungen:
- EP-A1- 2 821 028
- WO-A2-2012/085285
- DE-A1-102009 056 752
- DE-A1-102013 003 913

## Beschreibung

Die Erfindung betrifft ein Verfahren zur Herstellung einer Wachsbasis oder einer Prothesenbasis zur Herstellung einer Dentalprothese, bei dem ein virtuelles CAD-Modell der Dentalprothese zur Herstellung der physischen Dentalprothese oder der physischen Prothesenbasis verwendet wird, wobei aus dem CAD-Modell der Dentalprothese ein virtuelles Modell der Prothesenbasis berechnet wird. Die Erfindung betrifft auch eine Wachsbasis oder Prothesenbasis, eine Wachsaufstellung und eine Dentalprothese hergestellt mit einem solchen Verfahren.

Der gängige Weg ist die analoge Erstellung von Dentalprothesen. Zur Herstellung der Prothesenbasis wird derzeit also meist ein analoges Verfahren verwendet, bei dem zunächst ein Abdruck des zahnlosen Kiefers des Patienten genommen wird. Aus diesem Abdruck wird ein Gipsmodell der Patientensituation gefertigt. Anschließend wird ein Funktionsmodell der Prothesenbasis aus Wachs (die Wachsbasis) auf dem Gipsmodell aufgebaut und mit Prothesenzähnen bestückt. Von dieser Aufstellung wird dann die Wachsbasis entfernt und die Aufstellung der künstlichen Zähne in eine finale Basis aus Kunststoff überführt. Dazu wird eine Küvette als Hohlform oder Gießform verwendet, mit der die mit Prothesenzähnen bestückte Wachsbasis eingebettet wird. Aus der ausgehärteten Gipsform wird das Wachs mit heißem Wasser ausgewaschen, so dass ein Hohlraum für den Prothesenkunststoff der Prothesenbasis entsteht. Durch die Vorgehensweise sind die Prothesenzähne bereits in die Hohlform eingesetzt. Die Form wird mit einem zahnfleischfarbenen Kunststoff ausgegossen oder gestopft und während des Gießprozesses werden die Prothesenzähne mit der Prothesenbasis verbunden. Nach Aushärtung des Kunststoffs wird dieser nachbearbeitet, um die gewünschte Form zu erhalten.

Zur Herstellung der Dentalprothese werden meist vorkonfektionierte Prothesenzähne manuell und einzeln auf der Wachsbasis auf einem Gipsmodell des unbezahnten Kiefers aufgestellt. Diese Wachsprothese wird im nächsten Schritt in die Küvette mit Gips, Silikon oder Gel (je nach späterer Verarbeitungstechnik) eingebettet, um dann nach Aushärten des Einbettmaterials die Wachsbasis aus der Form heraus zu lösen, um den Hohlraum für den Prothesenkunststoff zu schaffen. Die Prothesenzähne verbleiben dabei im Einbettmaterial. Ein entsprechender Kunststoff wird in den Hohlraum injiziert oder gegossen, dadurch erhält man nach Aushärtung des Kunststoffs die Dentalprothese. Bei der Aufstellung der Prothesenzähne werden diese der jeweiligen Mundsituation des Patienten vom Zahntechniker und gegebenenfalls auch vom Zahnarzt angepasst und beschliffen.

Das gesamte derartige Verfahren ist sehr Zeit- und damit Kostenintensiv und basiert auf einer großen Zahl handwerklicher Schritte. Im Zuge der Digitalisierung verlagert sich der Aufwand zur Herstellung, Aufstellung und Konstruktion der Prothesenbasen und der Prothesenzähne in das CAD-Modell und die eigentliche Herstellung wird von Maschinen (CAM-Maschinen) übernommen.

Digitale Fertigungsmethoden gewinnen im Dentalbereich immer mehr an Bedeutung. Zahnersatz, wie zum Beispiel Kronen und Brücken, wird seit einigen Jahren mittels CAD/CAM-Technologien subtraktiv in Fräsverfahren hergestellt (CAM - Computer-Aided Manufacturing, Deutsch: rechner-unterstützte Fertigung, CAD - Computer-Aided Design, Deutsch: rechnerunterstützte Konstruktion). Die Problematik der Verbindung inklusive der Korrekturmöglichkeit besteht jedoch in der herkömmlichen wie auch in der digitalen Vorgehensweise.

Die DE 10 2013 003 913 A1 offenbart ein Verfahren zur Herstellung einer Prothesenbasis, bei dem auf Basis eines digitalen Datensatzes zunächst ein Wachsprovisorium mit Ausnehmungen zur Aufnahme von künstlichen Zähnen hergestellt wird und anschließend unter Anwendung des Wachsprovisoriums die eigentliche Prothesenbasis angefertigt wird.Ein CAD/CAM-Verfahren zur Herstellung einer Dentalprothese ist aus der WO 91/07141 A1 bekannt, wobei bei diesem Verfahren eine Prothesenbasis auf der Basis eines Abdrucks aus einem Kunststoffblock (einer sogenannten Ronde) gefräst wird. Die herausgefräste Prothesenbasis ist lediglich 2 bis 3 mm dick.

Nachteilig ist also eine sehr hohe Materialverschwendung, die je nach anatomischer Situation des jeweiligen Patienten auch über 90% betragen kann. Zudem erfordert das Herausfräsen derart großer Materialmengen entsprechend viel Zeit und belastet die Fräswerkzeuge, so dass sich diese schneller verbrauchen und getauscht werden müssen. Des Weiteren kann das Wissen und die handwerklichen Fähigkeiten von Zahntechnikern nicht mehr oder nur schlecht genutzt werden, wenn alle Modellierungsschritte ausschließlich am Computer zur Erzeugung des CAD-Modells erfolgen.

Die Aufgabe der Erfindung besteht also darin, die Nachteile des Stands der Technik zu überwinden. Insbesondere soll ein Verfahren bereitgestellt werden, mit dem eine aufwendige und zeitintensive Bearbeitung mit Fräsen vermieden wird. Zudem sollen die Fräswerkzeuge geschont werden und eine schnelle Fertigung ermöglicht werden.

Ferner soll eine möglichst einfache, vollständige und kostengünstige Herstellung der Wachsbasis, der Prothesenbasis und der Dentalprothese möglich sein. Des Weiteren ist es Aufgabe der Erfindung, einen Weg aufzuzeigen, mit dem die bekannten Methoden der Dentalprothesenherstellung möglichst gut mit modernen Fertigungsverfahren kombiniert werden können, um das bei der herkömmlichen Herstellung von Dentalprothesen vorhandene Knowhow auch in Kombination mit modernen CAD/CAM-Verfahren nutzbar zu machen. Mit der Erfindung soll auch eine Wachsbasis, eine Prothesenbasis, eine Wachsaufstellung und eine fertige Dentalprothese bereitgestellt werden. Des Weiteren soll es erfindungsgemäß auch ermöglicht werden, Unterstrukturen wie Implantate, Stege, Verankerungsvorrichtungen und/oder Befestigungsmittel zur Befestigung einer Hybridprothese an solchen Implantaten, Stegen und/oder Verankerungsvorrichtungen bei der Herstellung zu integrieren beziehungsweise diese zu berücksichtigen.

Die Aufgaben der Erfindung werden gelöst durch ein Verfahren zur Herstellung einer Wachsbasis oder einer Prothesenbasis zur Herstellung einer Dentalprothese, bei dem ein virtuelles CAD-Modell der Dentalprothese zur Herstellung der physischen Dentalprothese oder der physischen Prothesenbasis verwendet wird, wobei aus dem CAD-Modell der Dentalprothese ein virtuelles Modell der Prothesenbasis berechnet wird, gekennzeichnet durch die folgenden chronologischen Schritte:
A) Einscannen zumindest eines dreidimensionalen Bereichs eines physischen Modells der Mundraumsituation eines Patienten und Erstellen eines Datensatzes D1 auf Basis dieses Scans oder
   Intraoralscan eines dreidimensionalen Bereichs der Mundraumsituation des Patienten und Herstellung eines physischen Modells der Mundraumsituation eines Patienten auf Basis des Intraoralscans und Erstellen eines Datensatzes D1 auf der Basis des Intraoralscans;
B) Aufbringen einer Wachsschicht oder einer Kunststoffschicht auf das physische Modell der Mundraumsituation;
D) Berechnung einer virtuellen Oberfläche als eine in der Wachsschicht oder in der Kunststoffschicht zu erzeugenden Oberfläche, wobei die Berechnung auf Basis des virtuellen Modells der Prothesenbasis und des Datensatzes D1 erfolgt und wobei in der zu erzeugenden Oberfläche Positionierhilfen zur Positionierung von Prothesenzähnen vorgesehen werden; und
E) Herstellen der Wachsbasis aus der Wachsschicht oder der Prothesenbasis aus der Kunststoffschicht mit einem subtraktiven CAM-Verfahren auf Basis der in Schritt D) berechneten virtuellen Oberfläche.

Ein Scan oder Intraoralscan im Sinne der vorliegenden Erfindung ist dabei eine 3D-Aufnahme der gescannten Oberfläche. Der Scan oder Intraoralscan kann beispielsweise durch eine Stereoaufnahme mit einer Stereokamera erfolgen.

Das physische Modell der Mundraumsituation umfasst bevorzugt ein Modell des Oberkiefers und des Unterkiefers sowie die Relation der beiden Modelle zueinander. Das Verfahren kann aber auch mit einem physischen Modell der Mundraumsituation durchgeführt werden, das nur ein Modell des Oberkiefers oder Unterkiefers oder von Teilen davon enthält.

Das virtuelle CAD-Modell der Dentalprothese wird vorzugsweise erzeugt, indem ein physisches Modell-Paar der Mundraumsituation des Oberkiefers und des Unterkiefers sowie deren Relation zueinander oder der Oberkiefer und der Unterkiefer der Mundraumsituation sowie deren Relation zueinander eingescannt beziehungsweise vermessen wird und anschließend mit einer CAD-Software die Dentalprothese als virtuelles CAD-Modell designt wird. Aus dem virtuellen CAD-Modell der Dentalprothese können durch rechnerische Trennung ein Datensatz für die Prothesenzähne und ein Datensatz für die Prothesenbasis erzeugt werden. Letzterer Datensatz für die Prothesenbasis ist dann das virtuelle Modell der Prothesenbasis. Das virtuelle Modell der Prothesenbasis kann anschließend verwendet werden, um eine physische Prothesenbasis aus Wachs oder Kunststoff zu erzeugen.

Erfindungsgemäß bevorzugt kann vorgesehen sein, dass die Wachsschicht oder die Kunststoffschicht eine Stärke von mindestens 3mm aufweist, bevorzugt eine Stärke zwischen 5 mm und 15 mm, besonders bevorzugt zwischen 8 mm und 12 mm.

Mit der Erfindung wird auch ein Schritt C) vorgeschlagen, der zwischen den Schritten B) und D) erfolgt:
C) Einscannen von zumindest einem Bereich des mit der Wachsschicht oder der Kunststoffschicht belegten physischen Modells der Mundraumsituation und Erstellen eines zweiten Datensatzes D2 auf Basis dieses Scans.

Hierdurch kann der Unterschied zwischen dem unbeschichteten und dem beschichteten Modell des Mundraums zur Steuerung des CAM-Verfahrens in Schritt E) verwendet werden und/oder zur Berechnung der virtuellen Oberfläche in Schritt D) verwendet werden.

Dabei kann vorgesehen sein, dass in Schritt C) die freie Oberfläche der Wachsschicht oder der Kunststoffschicht auf dem physischen Modell des Mundraums zumindest bereichsweise eingescannt wird und der Scan zu dem zweiten Datensatz D2 verarbeitet wird, wobei bei der Berechnung der virtuellen Oberfläche als die zu erzeugende Oberfläche in der Wachsschicht oder in der Kunststoffschicht auf Basis des CAD-Modells der Prothesenbasis, auf Basis des ersten Datensatzes D1 und auf Basis des zweiten Datensatzes D2 erfolgt.

Hierdurch ist die Oberfläche der Wachsschicht oder der Kunststoffschicht bekannt, so dass beim anschließenden subtraktiven CAM-Verfahren nur in den Bereichen abgetragen wird, beziehungsweise abgetragen werden muss, in denen auch wirklich Material (Wachs oder Kunststoff) zum Abtragen vorhanden ist. Dadurch kann die Fertigung beschleunigt werden, da aus den virtuellen Modellen der Wachsschicht oder der Kunststoffschicht bekannt ist, an welchen Stellen sich Wachs oder Kunststoff befindet. Bevorzugt wird zusätzlich auch ein Teil des physischen Modells des Mundraums mit eingescannt, um die exakte Positionierung der Wachs- oder Kunststoffschicht auf dem physischen Modell des Mundraums zu bestimmen beziehungsweise sicherzustellen.

Gemäß einer bevorzugten Weiterbildung des erfindungsgemäßen Verfahrens kann vorgesehen sein, dass die Oberfläche, die in der Wachsschicht oder der Kunststoffschicht erzeugt wird, der Auflagefläche der Wachsschicht oder der Kunststoffschicht auf dem physischen Modell des Mundraums gegenüber liegt.

Damit kann die Berechnung und Herstellung der Oberfläche auf die wesentlichen und zugänglichen Teile beschränkt werden.

Des Weiteren kann vorgesehen sein, dass die Positionierhilfen eine eindeutige Form derart aufweisen, dass die Lage und die Position der Prothesenzähne zueinander eindeutig vorgegeben wird, wenn die Prothesenzähne flächenschlüssig in die Positionierhilfen der Prothesenbasis eingesetzt sind oder wenn die Prothesenzähne flächenschlüssig in die Positionierhilfen der Wachsbasis eingesetzt sind.

Bevorzugt kann erfindungsgemäß vorgesehen sein, dass die Positionierhilfen als Zahnfächer ausgeführt sind. Zahnfächer sind Vertiefungen in der Prothesenbasis oder der Wachsbasis, mit einer zur Aufnahme der Prothesenzähne passenden Form.

Dazu weisen die Zahnfächer besonders bevorzugt neben einem Boden auch eine umlaufende Wandung auf. Die Prothesenzähne werden dann einfach basal in die Wachsprothese eingesteckt oder basal in die Prothesenbasis eingeklebt.

Als Positionierhilfen können als Alternative zu Zahnfächern auch Erhebungen auf der Wachsbasis oder der Prothesenbasis vorgesehen sein, auf die die Prothesenzähne gesteckt werden. Solche Erhebungen zum Realisieren der Positionierhilfen können beispielsweise durch zylindrische Erhebungen realisiert werden, nach Art wie diese auch auf Lego®-Steinen vorgesehen sind (Lego®-Stein-Prinzip). Die Erhebungen beziehungsweise die Positionierhilfen können aber auch bevorzugt eine nicht drehsymmetrische Symmetrie aufweisen, um eine eindeutige axiale Positionierung der Prothesenzähne zu ermöglichen. Zudem können die Größe und die Höhe der Erhebungen beziehungsweise allgemeiner die Form der Positionierhilfen (auch wenn es keine Erhebungen sind) für jeden Prothesenzahn eindeutig sein, um eine eindeutige Positionierung und Ausrichtung der Prothesenzähne sicherzustellen.

Durch eindeutig geformte Positionierhilfen kann eine eindeutige Positionierung der Prothesenzähne sichergestellt werden, so dass die Gefahr einer Fehlsetzung der Prothesenzähne reduziert oder vermieden wird. Dies beugt einer fehlerhaften Fertigung der Dentalprothese vor. Bevorzugt befindet sich die Wachsbasis oder die Prothesenbasis auf dem physischen Modell der Mundraumsituation, während die Prothesenzähne in die Positionierhilfen eingesetzt werden.

Besonders bevorzugte Ausgestaltungen der vorliegenden Erfindung können vorsehen, dass die Herstellung der Wachsbasis aus der Wachsschicht oder die Herstellung der Prothesenbasis aus der Kunststoffschicht mit einem subtraktiven CAM-Verfahren erfolgt, während die Wachsschicht oder die Kunststoffschicht auf dem physischen Modell des Mundraums angeordnet ist.

Die Unterlage, die das physische Modell des Mundraums bietet, kann besonders gut zur Stabilisierung und Positionierung der Wachsbasis oder der Prothesenbasis während der Bearbeitung eingesetzt werden.

Es ist erfindungsgemäß bevorzugt, wenn beim Einscannen des zumindest einen Bereichs des physischen Modells der Mundraumsituation in Schritt A) zumindest die Oberflächen eingescannt werden, auf die die Wachsschicht oder die Kunststoffschicht anschließend aufgebracht wird oder beim Intraoralscan der Mundraumsituation des Patienten in Schritt A) zumindest die Oberflächen eingescannt werden, auf die die Wachsschicht oder die Kunststoffschicht bei dem physischen Modell aufgebracht wird.

Hierdurch wird sichergestellt, dass die gesamte Unterseite beziehungsweise Auflagefläche der Wachsschicht oder der Kunststoffschicht beim Durchführen der nachfolgenden Berechnungen bekannt ist.

Ferner kann zur Standardisierung, Reproduzierbarkeit und Vereinfachung des Verfahrens vorgesehen sein, dass das Aufbringen der Wachsschicht oder der Kunststoffschicht auf das physische Modell der Mundraumsituation mit Hilfe vorgeformter Wachskörper, insbesondere mit Hilfe von zumindest einer Wachsplatte, oder mit Hilfe weicher vorgeformter Kunststoffkörper, insbesondere mit Hilfe von zumindest einer Kunststoffplatte, durchgeführt wird und/oder ein Abformlöffel zum Aufbringen auf dem physischen Modell des Mundraums verwendet wird.

Das Wachs oder der Kunststoff können zuvor beispielsweise durch Erwärmen erweicht worden sein. Unter einer weichen Kunststoffplatte oder einer weichen Kunststoffkörper wird ein solcher verstanden, dessen plastische Verformbarkeit ausreicht, um bei manueller Krafteinwirkung den Kunststoff an die gewünschte Oberfläche des physischen Modells der Mundraumsituation zu drücken. Hierzu können auch einfache Werkzeuge eingesetzt werden. Bevorzugt kann eine Mehrzahl verschiedener Abformlöffel, die für unterschiedliche Typen, beziehungsweise Größenbereiche von Mundraumsituationen passend sind, verwendet werden, um die Wachsschicht oder die Kunststoffschicht auf dem physischen Modell der Mundraumsituation aufzutragen.

Hierdurch kann der Vorgang des Auftragens für den Anwender stark vereinfacht werden. Zudem können so mögliche Fehler bei der Beschichtung, wie sie beispielsweise durch Lücken oder schlechte Verbindungen verursacht werden können, besser verhindert werden.

Mit einer Weiterbildung wird vorgeschlagen, dass das physische Modell des Mundraums vor Schritt A) durch einen Abdruck des Oberkiefers und/oder einen Abdruck des Oberkiefers im Mundraum des Patienten gewonnen wird, wobei vorzugsweise der Abdruck mit Gips oder einem Kunststoff ausgegossen wird, um ein Gips-Modell oder ein Kunststoff-Modell als physisches Modell der Mundraumsituation zu erzeugen.

Hierdurch werden bekannte und erprobte Verfahren zur Umsetzung des erfindungsgemäßen Verfahrens in Anwendung gebracht. Bevorzugt wird ein Modell-Paar (Ober- und Unterkiefer) aus Gips in Relation zueinander als physisches Modell der Mundraumsituation hergestellt. Die Relation des Modell-Paars kann dabei durch die Verwendung eines Artikulators eingestellt werden.

Besonders vorteilhaft sind erfindungsgemäße Verfahren, bei denen vorgesehen ist, dass das virtuelle CAD-Modell der Dentalprothese und/oder das virtuelle Modell der Prothesenbasis auf Basis des Datensatzes D1 des Scans des physischen Modells der Mundraumsituation oder des Intraoralscans der Mundraumsituation durch ein CAD-Verfahren erzeugt wird.

Im ersten Fall wird das für das Verfahren ohnehin vorgesehene und verwendete physische Modell der Mundraumsituation auch gleichzeitig zur Berechnung des virtuellen Modells der Dentalprothesen verwendet. Zudem können so Abweichungen zwischen dem virtuellen CAD-Modell der Dentalprothese und der zu erzeugenden Wachsbasis oder Prothesenbasis vermieden werden.

Des Weiteren kann vorgesehen sein, dass zur Umsetzung des subtraktiven CAM-Verfahrens eine computergesteuerte Fräse verwendet wird, bevorzugt eine computergesteuerte 4-Achs- oder 5-Achs-Fräse.

Mit diesen Fräsen kann das Verfahren weiter automatisiert und schnell durchgeführt werden.

Wenn eine Hybridprothese aus der Wachsbasis oder der Prothesenbasis aus Kunststoff gefertigt werden soll, kann erfindungsgemäß vorgesehen sein, dass zur Herstellung der Wachsbasis oder der Prothesenbasis zumindest ein Befestigungsmittel an dem physischen Modell der Mundraumsituation angeordnet wird, insbesondere an den Stellen des physischen Modells der Mundraumsituation angeordnet wird, die Implantaten entsprechen, oder zumindest ein Befestigungsmittel an einer Schiene und/oder einer Verankerungsvorrichtung befestigt wird, die an dem physischen Modell der Mundraumsituation angeordnet wird oder werden, insbesondere an den Stellen des physischen Modells der Mundraumsituation angeordnet wird oder werden, die Implantaten entsprechen, und anschließend in Schritt B) die Wachsschicht oder die Kunststoffschicht derart auf das physische Modell der Mundraumsituation aufgebracht wird, dass die Wachsschicht oder die Kunststoffschicht mit dem zumindest einen Befestigungsmittel verbunden ist.

Hierdurch lässt sich die herzustellende Prothesenbasis gleich mit dem zumindest einen Befestigungsmittel verbunden erzeugen. Mit dem erfindungsgemäßen Verfahren lässt sich dann also auch eine Hybridprothese fertigen. Implantate, Stege oder sonstige Verankerungsvorrichtungen für die Dentalprothese können so mit dem erfindungsgemäßen Verfahren berücksichtigt werden.

Bevorzugt kann dabei vorgesehen sein, dass der Scan des physischen Modells der Mundraumsituation oder der Intraoralscan mit Implantaten, mit zumindest einem eingesetzten Steg und/oder mit zumindest einer Verankerungsvorrichtung am physischen Modell oder im Mundraum durchgeführt wird, besonders bevorzugt mit zumindest einem an den Implantaten, an dem zumindest einen eingesetzten Steg und/oder an der zumindest einen Verankerungsvorrichtung befestigten Befestigungsmittel.

Hierdurch können jegliche Unterstrukturen (Implantate, Schiene, Verankerungsvorrichtungen, Befestigungsmittel) unter der Wachsbasis oder Kunststoffbasis mit dem Scan oder dem Intraoralscan erfasst werden und bei der Berechnung der virtuellen Oberfläche in Schritt D) berücksichtigt werden.

Die der vorliegenden Erfindung zugrundeliegenden Aufgaben werden auch gelöst, durch ein Verfahren zur Herstellung einer Dentalprothese oder einer Wachsaufstellung für eine Dentalprothese mit einem vorangehend beschriebenen erfindungsgemäßen Verfahren wobei nach Schritt E) ein Schritt F) erfolgt:
F) Einsetzen von Prothesenzähnen in die Positionierhilfen der Wachsbasis, die in Schritt E) in der Oberfläche der Wachsschicht erzeugt wurden, zur Herstellung der Wachsaufstellung, oder Einsetzen und Befestigen, insbesondere Einkleben, von Prothesenzähnen in die Positionierhilfen der Prothesenbasis, die in Schritt E) in der Oberfläche der Kunststoffschicht erzeugt wurden, zur Herstellung der Dentalprothese, wobei bevorzugt die Dentalprothese nach dem Einsetzen und Befestigen der Prothesenzähne poliert wird und/oder die Verbindung der Prothesenzähne zur Prothesenbasis ausgehärtet wird.

Hierdurch kann die physische Wachsaufstellung beziehungsweise die physische Dentalprothese auf einfache Weise fertiggestellt werden.

Des Weiteren werden die der vorliegenden Erfindung zugrundeliegenden Aufgaben gelöst durch ein Verfahren zur Herstellung einer Dentalprothese aus einer Wachsaufstellung, hergestellt mit einem solchen Verfahren, wobei nach Schritt F) die Wachsaufstellung mit den Prothesenzähnen in eine Form eingegossen wird, das Wachs der Wachsbasis entfernt wird, insbesondere durch Schmelzen des Wachses und durch Herauslösen des Wachses mit Wasserdampf, wobei die Prothesenzähne in der Form verbleiben und die Lage und Position der Prothesenzähne in der Form unverändert bleiben, das Wachs der Wachsbasis entfernt wird, insbesondere durch Schmelzen des Wachses und durch Herauslösen des Wachses mit Wasserdampf, wobei die Prothesenzähne in der Form verbleiben und die Lage und Position der Prothesenzähne in der Form unverändert bleiben, der in der Form entstandene Hohlraum wird mit einem Kunststoff ausgegossen, der die Prothesenbasis der Dentalprothesen bildet, der Kunststoff wird ausgehärtet, wobei die Prothesenzähne mit dem Kunststoff fest verbunden werden, die Form wird geöffnet und die Dentalprothese aus der Form entnommen, wobei vorzugsweise die Dentalprothese nach dem Entnehmen aus der Form durch Politur und/oder Entgratung nachbearbeitet wird.

Hierdurch können die in Dentallabors heutzutage üblichen Herstellungsverfahren mit modernen rechnergestützten Verfahren kombiniert werden, so dass sowohl das bereits vorhandene Knowhow der Zahntechniker als auch moderne Erkenntnisse zur Optimierung der Herstellung von Dentalprothesen genutzt werden können.

Die Aufgaben der vorliegenden Erfindung werden auch gelöst durch eine Wachsbasis oder Prothesenbasis hergestellt mit einem solchen Verfahren und durch eine Dentalprothese hergestellt mit einem solchen Verfahren.

Die Prothesenzähne bestehen bevorzugt aus einem Kunststoff, besonders bevorzugt aus Polymethylmethacrylat (PMMA).

Die Prothesenzähne können einzeln und/oder in mehreren Gruppen zusammenhängend oder als komplette Zahnreihen zusammenhängend vorliegen. Zusammenhängende Prothesenzähne sind fest miteinander verbunden.

Der Kunststoff zur Herstellung der Prothesenbasis besteht bevorzugt aus einem rosa-farbenen oder pinken Kunststoff und die Prothesenzähne aus einem zahnfarbenen Kunststoff.

Der Erfindung liegt die überraschende Erkenntnis zugrunde, dass es mit der vorliegenden Erfindung gelingt, ein Material-sparendes Verfahren anzugeben, mit dem eine Prothesenbasis, eine Wachsbasis beziehungsweise eine Dentalprothese hergestellt werden kann, wobei gleichzeitig moderne CAD/CAM-Verfahren genutzt werden, so dass einerseits die Vorteile, wie die Automatisierung, der CAD/CAM-Verfahren genutzt werden können, gleichzeitig aber eine schnell umzusetzende und Material-sparende und Werkzeug-schonende Fertigung ermöglicht wird. Gleichzeitig kann das in zahntechnischen Labors vorhandene Wissen und handwerkliche Geschick nutzbringend eingesetzt werden, insbesondere dann, wenn die Prothesenzähne zunächst auf einer Wachsbasis aufgestellt werden und die eigentliche Dentalprothese anschließend mit einer darauf basierenden Form mit Hilfe einer Küvette gefertigt wird oder wenn die Kunststoffschicht oder die Wachsschicht manuell, mit dem verständigen Wissen eines Zahntechnikers aufgetragen wird, der Einzuschätzen vermag, an welchen Stellen des physischen Modells des Mundraums Material für die Prothesenbasis beziehungsweise Wachsbasis aufzutragen ist und an welchen Stellen kein Material vorhanden sein sollte und in welcher Stärke Material sinnvollerweise aufzutragen ist. Mit der Erfindung werden unter anderem die Vorteile erreicht, dass geringere Fräszeiten notwendig sind, dass ein geringerer Materialeinsatz an Basismaterial (Wachs beziehungsweise Kunststoff) notwendig ist und dass eine Schonung der Fräswerkzeuge (geringerer Verschleiß der Fräsköpfe und des Antriebs) möglich ist. Zudem kann eine größere Anzahl von Prothesenbasen, Wachsbasen und Dentalprothesen in der gleichen Zeit gefertigt werden, was bei den hohen Kosten, die eine CAM-Fräse in der Anschaffung verursacht, Vorteile bringt. Bei der Anwendung eines Intraoralscans kann die Auflagefläche für das virtuelle Modell der Wachsbasis oder der Kunststoffbasis direkt am Patienten aufgenommen werden beziehungsweise aus einer solchen Aufnahme ermittelt werden, so dass das physische Modell des Mundraums nicht mehr eingescannt werden muss, da es ja aus diesen Daten hergestellt wurde. Mögliche Fehler beim Nehmen eines Abdrucks entfallen so.

Zudem können mit dem erfindungsgemäßen Verfahren auch Hybridprothesen gefertigt werden. Hybridprothesen sind Dentalprothesen, die mit im Kiefer eingesetzten Implantaten verbunden werden. Dazu können mit den Implantaten Stege verbunden sein, die mit den Implantaten verbunden bleiben, wobei die Dentalprothese über mehrere Clips als Befestigungsmittel mit dem Steg verbunden werden können. Andere Verankerungsvorrichtungen zur Befestigung von Hybridprothesen sind denkbar und mit dem erfindungsgemäßen Verfahren anwendbar.

Der dieser Erfindung zu Grunde liegende Lösungsansatz geht von einer nur einseitigen Bearbeitung der Wachsbasis oder Kunststoffbasis aus, die besonders bevorzugt direkt auf dem physischen Modell der Mundraumsituation erfolgt.

Ein beispielhaftes erfindungsgemäßes Verfahren kann die folgenden Verfahrensschritte aufweisen:
A) Einscannen eines reinen Gipsmodells als physisches Modell der Mundraumsituation eines Patienten. Dabei ist insbesondere die Auflagefläche für die spätere Dentalprothese von Bedeutung. Hierbei spielt es keine Rolle, ob das physische Modell ausschließlich die reine Schleimhaut darstellt oder aber die Schleimhaut und bereits integrierte Prothetikelemente wie zum Beispiel Stege oder Implantataufbauten umfasst. Auch können noch vorhandene Zähne in dem physischen Modell dargestellt sein, wobei dann eine Teilprothese als Dentalprothese gefertigt wird.
B) Nach dem Einscannen wird eine Wachsschicht oder Kunststoffschicht auf das physische Modell adaptiert. Die Wachsschicht oder Kunststoffschicht sollte eine Stärke von mindestens 3 mm aufweisen, bevorzugt aber etwa 10 mm.
C) Das physische Modell des Mundraums mit der Wachs- oder Kunststoffschicht wird nun wieder eingescannt. Dabei ist insbesondere die Wachsbasis von Bedeutung. Dieser Schritt C) kann auch weggelassen werden, ist aber vorteilhaft, da so bei dem anschließenden subtraktiven CAM-Verfahren bekannt ist, wo die Oberfläche der Wachsschicht oder der Kunststoffschicht beginnt und damit der Fräskopf oder das Werkzeug gleich richtig gesteuert beziehungsweise gleich in die richtige Position gebracht werden kann, um das Material abzutragen. Hierdurch kann wieder Zeit bei der Herstellung der Wachsbasis oder der Prothesenbasis aus Kunststoff in der CAM-Vorrichtung gespart werden.
D) Nun erfolgt mit einer geeigneten Software ein Abgleich zwischen dem physischen Modell ohne Wachsschicht oder Kunststoffschicht und dem Modell mit Wachsschicht oder Kunststoffschicht. Zudem wird anhand eines virtuellen Modells der Prothesenbasis die in der Wachsschicht oder der Kunststoffschicht zu erzeugende Oberfläche berechnet. Dabei kann der Scan des physischen Modells des Mundraums als Unterseite beziehungsweise Auflagefläche für das virtuelle Modell der Dentalprothese und damit der Prothesenbasis oder der Wachsaufstellung (Wachsbasis mit Prothesenzähnen) verwendet werden. In das virtuelle Modell der Prothesenbasis werden Zahnfächer als Positionierhilfen zur Aufnahme von vorkonfektionierten Prothesenzähnen eingerechnet. Der Unterschied zwischen dem virtuellen Modell der Prothesenbasis und dem Scan des physischen Modells des Mundraums mit der Wachsschicht oder der Kunststoffschicht ergibt das mit dem CAM-Verfahren anschließend abzutragenden Volumenkörper (einschließlich der Form des Volumenkörpers). Damit wird also bestimmt, welcher Bereich der Wachsschicht oder der Kunststoffschicht abgetragen wird.
E) Mittels Fräskörpern wird die Wachsschicht oder Kunststoffschicht entsprechend der Patientensituation und der Prothesenzahnaufstellung nur an der Oberseite abgetragen. Ein maschinelles Bearbeiten der Unterseite zur Schleimhaut hin entfällt völlig. Die zuvor berechnete Position der Prothesenzähne wird entsprechend eingefräst.
F) Die Prothesenzähne werden in die Zahnfächer beziehungsweise die Positionierhilfen eingesetzt. Die fertige Wachsbasis oder Dentalprothese wird vom physischen Modell entnommen und zur Einprobe beim Patienten verwendet.

Das Verfahren lässt sich in ähnlicher Weise umsetzen, wenn zunächst die Mundraumsituation am Patienten mit einem Intraoralscan aufgenommen wird und das physische Modell der Mundraumsituation auf Basis dieses Intraoralscans hergestellt wird. Gegebenenfalls müssen Aufbauten wie Implantate oder Stege, die im Mundraum des Patienten eingesetzt sind, durch das physische Modell realisiert werden oder die Aufbauten (insbesondere bei Befestigungselementen) werden an dem physischen Modell angeordnet, um sie in die Wachsschicht oder Kunststoffschicht zu integrieren. Hierfür kann es auch vorteilhaft sein, wenn ein zusätzlicher Scan des physischen Modells der Mundraumsituation mit angelegten oder befestigten Befestigungselementen erfolgt und als Datensatz D1 verwendet wird.

Im Folgenden werden weitere Ausführungsbeispiele der Erfindung anhand von fünf schematisch dargestellten Figuren erläutert, ohne jedoch dabei die Erfindung zu beschränken. Dabei zeigt:
Figur 1: eine perspektivische Ansicht einer Prothesenbasis oder Wachsbasis für einen Unterkiefer ohne Prothesenzähne, wie sie sich aus dem virtuellen Modell der Prothesenbasis ergibt und wie sie sich beim Durchführen eines erfindungsgemäßen Verfahrens ergibt; und
die Figuren 2 bis 5: den chronologischen Ablauf eines erfindungsgemäßen Verfahrens anhand eines im Querschnitt schematisch dargestellten physischen Modells der Mundraumsituation auf das eine Prothesenbasis oder Wachsbasis aufgebaut wird.

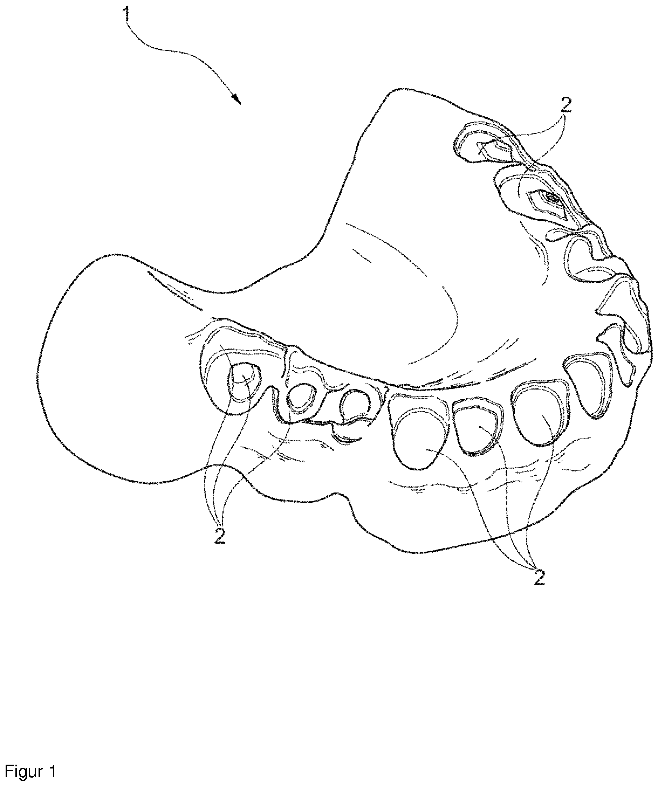

Figur 1 zeigt eine schematische perspektivische Ansicht einer Wachsbasis 1 oder einer Prothesenbasis 1 aus Kunststoff hergestellt mit einem erfindungsgemäßen Verfahren und vorgesehen zur Herstellung einer erfindungsgemäßen Wachsaufstellung oder einer erfindungsgemäßen Dentalprothese. Ebenso kann in Figur 1 auch das virtuelle Modell der Prothesenbasis dargestellt sein. Wenn die Prothesenbasis 1 aus Kunststoff gefertigt ist, besteht sie aus einem rosafarben eingefärbten Kunststoff. Die Färbung und Transparenz wird passend zu einer Zahnfleischoptik gewählt.

In der Oberseite der Wachsbasis 1 oder Prothesenbasis 1 sind eine Vielzahl von Zahnfächern 2 zur Fixierung von Prothesenzähnen (in Figur 1 nicht gezeigt) vorgesehen. Die Zahnfächer 2 sind dabei als Vertiefungen in dem Kiefersattel der Wachsbasis 1 oder Prothesenbasis 1 ausgeformt. Die Zahnfächer 2 können eine Indexierung aufweisen, so dass die Prothesenzähne nur in einem bestimmten Orientierungsbereich in der Wachsbasis 1 oder der Prothesenbasis 1 einzusetzen sind und jeder Prothesenzahn möglichst nur zu einem bestimmten Zahnfach 2 passt.

Die Zahnfächer 2 weisen in den Vertiefungen zentral angeordnete Erhebungen auf, die zu entsprechenden Vertiefungen in den basalen Enden der Prothesenzähne (in Figur 1 nicht gezeigt) passen. Dadurch kann noch eindeutiger und erkennbarer vorgegeben werden, welche Prothesenzähne in welche Zahnfächer 2 gesetzt werden sollen und in welcher Orientierung die Prothesenzähne in die Zahnfächer 2 gesteckt werden sollen. Die Erhebungen können nämlich bei geeigneter Symmetriebrechung als Indexierungen dienen. Zudem kann so eine stabilere Verbindung der Wachsbasis 1 oder der Prothesenbasis 1 zu den Prothesenzähnen erzeugt werden. Dies ist auch bei der Wachsbasis 1 von Bedeutung, da die mit Hilfe der Wachsbasis später erzeugte Prothesenbasis die gleiche Form wie die Wachsbasis 1 hat und spätestens beim Herstellen der Prothesenbasis eine stabile Verbindung zu den Prothesenzähnen bei der Fertigung der Dentalprothese gewünscht ist.

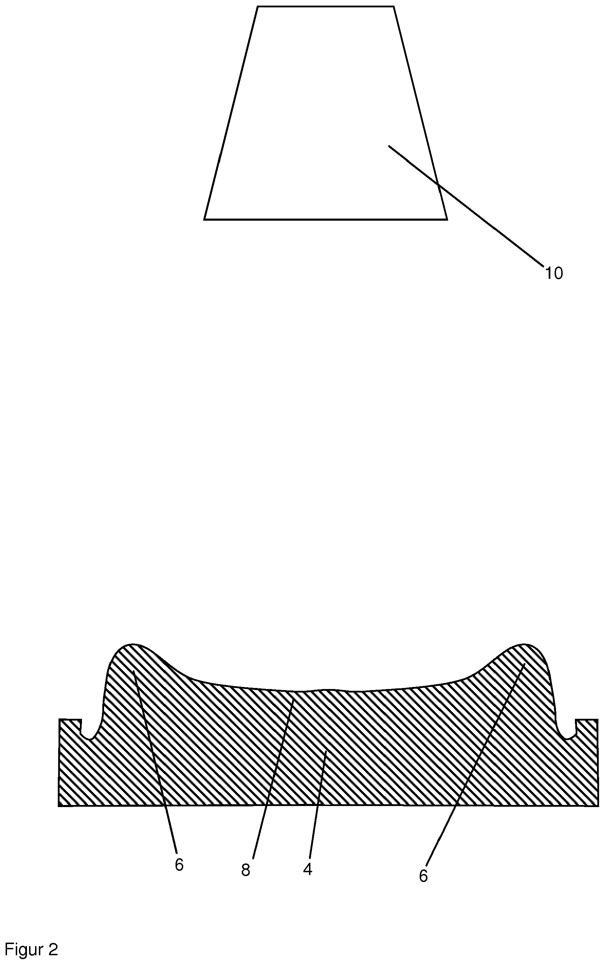

Die Figuren 2 bis 5 zeigen den chronologischen Ablauf eines erfindungsgemäßen Verfahrens anhand eines im Querschnitt schematisch dargestellten physischen Modells der Mundraumsituation auf das eine Prothesenbasis oder Wachsbasis und später eine Wachsaufstellung oder eine Dentalprothese aufgebaut wird. Die dargestellte Querschnittebene liegt in einer Ebene parallel zur Frontalebene des Patienten.

In Figur 2 ist das Einscannen eines physischen Modells 4 eines zahnlosen Oberkiefers eines Patienten dargestellt. Das physische Modell 4 wurde zuvor mit Hilfe einer Abformasse und eines Abformlöffels und Ausgießen der geformten Abformmasse erzeugt. Bevorzugt wird die geformte Abformmasse mit Gips ausgegossen, so dass ein Gipsmodell 4 erhalten wird. In dem physischen Modell 4 sind der Kieferbogen 6 beziehungsweise der Kieferkamm 6 und der Gaumen 8 der Mundraumsituation des Patienten erkennbar.

Das physische Modell 4 wird in einer definierten Position aufgestellt beziehungsweise fixiert und anschließend wird mit einem Scanner 10 die Oberfläche des physischen Modells 4 aufgenommen. Dazu kann der Scanner 10 und/oder das Modell 4 relativ zueinander bewegt werden, so dass alle relevanten Bereiche der Oberfläche des Modells 4, also insbesondere die Flächen, auf denen später die Prothesenbasis oder die Wachsbasis beziehungsweise die Dentalprothese aufliegen soll, aufgenommen werden können. Die Daten D1, die sich aus dem Scan ergeben, werden gespeichert.

Alternativ kann auch die Mundraumsituation des Patienten mit Hilfe eines Intraoralscans aufgenommen werden. Der Datensatz D1 wird dann aus dem Intraoralscan berechnet beziehungsweise auf dessen Basis ermittel. Dabei können auch offensichtliche Fehler im Modell, die beispielsweise auf einem Bildschirm graphisch durch den Benutzer erkennbar sind, durch den Benutzer manuell entfernt werden. Die Daten des Intraoralscans werden dann zusätzlich verwendet, um ein virtuelles CAD-Modell der Mundraumsituation und eines zu erzeugenden physischen Modells 4 der Mundraumsituation zu erzeugen. Mit einem geeigneten CAM-Verfahren wird dann generativ oder subtraktiv das physische Modell 4 der Mundraumsituation hergestellt.

Die Daten D1, unabhängig davon ob sie auf Basis eines Scans des Modells 4 oder auf Basis des Intraoralscans gewonnen wurden, können anschließend genutzt werden, um das virtuelle Modell der herzustellenden Dentalprothese mit einem CAD-Verfahren zu erzeugen. Dazu werden auch die Daten eines physischen Modells des Unterkiefers (in den Figuren 2 bis 5 nicht gezeigt), Daten zur Artikulation des Oberkiefers und des Unterkiefers des Patienten und Daten zur Form von einzusetzenden Prothesenzähne (also virtuelle Modelle von Prothesenzähnen) benötigt und verwendet. Durch rechnerisches Abziehen beziehungsweise rechnerische Trennung der Formen der Prothesenzähne von dem CAD-Modell ergibt sich ein virtuelles Modell der Prothesenbasis.

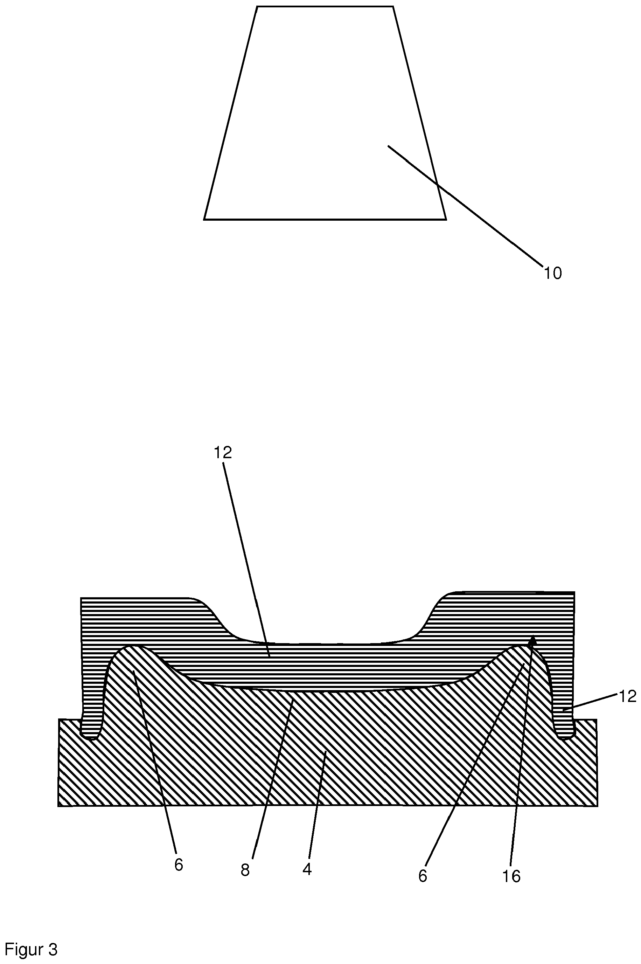

Anschließend wird eine Wachsschicht 12 oder eine Kunststoffschicht 12 auf das physische Modell 4 aufgetragen, das heißt, eine Basisplatte 12 aus Wachs oder Kunststoff auf dem physischen Modell 4 adaptiert. Dies ist in Figur 3 dargestellt. Die Wachsschicht 12 oder Kunststoffschicht 12 hat eine Stärke von 10 mm und ist damit in jedem Fall dicker als die zu erzeugende Wachsbasis 1 oder Prothesenbasis 1 an ihrer dicksten Stelle. Dadurch ist sichergestellt, dass später kein Material an irgendeiner Stelle der zu erzeugenden Wachsbasis 1 oder Prothesenbasis 1 fehlt.

Die Wachsschicht 12 oder die Kunststoffschicht 12 liegt auf dem Kieferbogen 6 oder Kieferkamm 6 und dem Gaumen 8 des physischen Modells 4 des Mundraums auf, das dem Anwender die relevanten anatomischen Strukturen des Mundraums des Patienten vorgibt. Das physische Modell 4 hält die Schicht 12 und später die daraus gefertigte Wachsbasis 1 oder Prothesenbasis 1 stabil. Zur Herstellung einer Prothesenbasis für den Unterkiefer wird ein entsprechendes Modell (nicht gezeigt) des Unterkiefers verwendet.

Mit dem Scanner 10 wird nun die Oberfläche des Modells 4 mit der Wachsschicht 12 oder Kunststoffschicht 12 darauf eingescannt (dargestellt in Figur 3). Dabei wird die gesamte Oberfläche der Wachsschicht 12 oder Kunststoffschicht 12 aufgenommen, die der Auflagefläche auf dem physischen Modell 4 gegenüberliegt. Dieser Scan wird als Datensatz D2 gespeichert.

Aus den Daten D1 der Oberfläche des physischen Modells 4, dem virtuellen Modell der Prothesenbasis und den Daten D2 der Oberfläche der Wachsschicht 12 oder der Kunststoffschicht 12 auf dem physischen Modell 4 wird dann berechnet, wie viel Material (Wachs oder Kunststoff) und an welchen Stellen das Material von der Wachsschicht 12 oder Kunststoffschicht 12 abgetragen werden muss und dieses Ergebnis als Datensatz D3 gespeichert.

Basierend auf den Daten D3 wird mit einer computergesteuerten Fräse 14 (beispielsweise einer computergesteuerten 4-Achsfräse oder 5-Achsfräse) die Wachschicht 12 oder Kunststoffschicht 12 bearbeitet beziehungsweise gefräst, während sie noch auf dem physischen Modell 4 aufliegt. Hierzu wird das physische Modell 4 der Mundraumsituation und die Wachsschicht 12 oder Kunststoffschicht 12 auf einer Halterung (nicht gezeigt) der computergesteuerten Fräse 14 befestigt und fixiert.

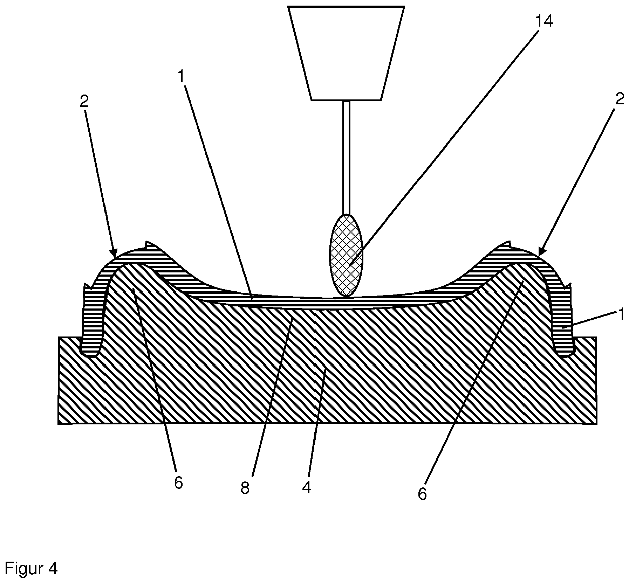

Die Bearbeitung in einem sehr weit fortgeschrittenen Stadium ist in Figur 4 dargestellt. Neben der gewünschten Form der so erzeugten Wachsbasis 1 oder Prothesenbasis 1 aus Kunststoff werden auch Zahnfächer 2 in der Wachsbasis 1 oder Prothesenbasis 1 erzeugt. Eine derartige fertige Wachsbasis 1 oder Prothesenbasis 1 mit den Zahnfächern 2 ist in Figur 1 dargestellt. Die auf dem physischen Modell 4 des Mundraums aufliegende Fläche muss nicht mit der Fräse bearbeitet werden und bleibt dadurch sehr glatt. Gegebenenfalls kann eine Politur auf dieser Seite erfolgen. Hierdurch kann entfällt die Bearbeitung einer Seite der Prothesenbasis 1 beziehungsweise der Wachsbasis 1, wodurch erheblich Zeit eingespart werden kann.

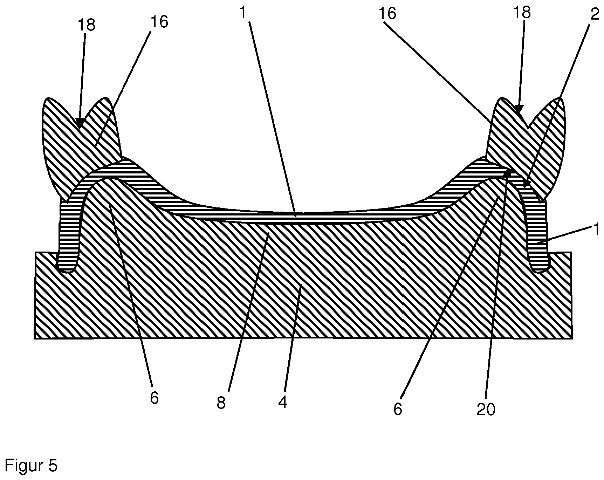

In einem nächsten Schritt werden Prothesenzähne 16 in die Zahnfächer 2 eingesetzt und darin befestigt. Dies ist in Figur 5 dargestellt.

Die geschnittenen Prothesenzähne 16 sind hier als Molaren ausgebildet beziehungsweise als Backenzahnprothesen ausgebildet. Die Prothesenzähne 16 bestehen aus einem harten weißen Kunststoff mit einer für Zähne oder zu den Zähnen des Patienten passenden Färbung und Transparenz. Jeder Prothesenzahn 16 weist eine koronale beziehungsweise okklusale Oberfläche 18 (Kaufläche) und eine basale Oberfläche 20 auf. Die basale Oberfläche 20 wird in den Zahnfächern 2 fixiert.

Die Zahnfächer 2 sind die basalen Gegenstücke der basalen Seiten 20 der Prothesenzähne 16. Vor dem ersten Einsetzen der Prothesenzähne 16 in die Zahnfächer 2 in die Prothesenbasis 1 aus Kunststoff, werden die Zahnfächer 2 und die Prothesenzähne 16 (zumindest die basalen Seiten 20 der Prothesenzähne 16) gereinigt und zur Verbesserung der Verbindung mit einem Lösungsmittel angerauht und angequollen und anschließend mit einem Klebstoff verbunden.

Hervorquellende überschüssige Reste des Klebstoffs können vor dem Aushärten und/oder nach dem Aushärten entfernt werden.

Die fertige Dentalprothese oder die Wachsaufstellung kann dann zur Anprobe beim Patienten eingesetzt werden. Die Wachsaufstellung (Wachsbasis 1 mit eingesetzten Prothesenzähnen 12) bietet noch weitreichende Möglichkeiten zur Korrektur durch den Zahntechniker und/oder den Zahnarzt. Basierend auf der Wachsaufstellung wird mit bekannten Verfahren mit Hilfe einer Küvette als Form und durch Entfernen des Wachses aus der Form eine Dentalprothese erzeugt, indem der Hohlraum mit den darin fixierten Prothesenzähnen 12 mit Kunststoff gefüllt wird und mit den Prothesenzähnen 12 verbunden wird. Eine Nachbearbeitung der fertigen Dentalprothese (Politur und Entgratung) kann anschließend sattfinden.

Das erfindungsgemäße Verfahren kann auch auf gedruckte Prothesenzähne oder Prothesenzahnreihen angewandt werden, wird aber bevorzugt mit vorkonfektionierten Prothesenzähnen ausgeführt, die zuvor manuell oder mit einem automatischen Fräsverfahren basal gekürzt werden können.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Wachsbasis / Prothesenbasis
- 2: Zahnfach zur Fixierung von Prothesenzähnen
- 4: Physisches Modell des Oberkiefers / Mundraums
- 6: Kieferbogen / Kieferkamm des Modells des Mundraums
- 8: Gaumen des Modells des Mundraums
- 10: Scanner
- 12: Wachsschicht / Kunststoffschicht
- 14: Fräse / Fräskopf
- 16: Prothesenzahn
- 18: Koronale Fläche des Prothesenzahns
- 20: Basale Fläche / basale Seite des Prothesenzahns

## Patentansprüche

1. Verfahren zur Herstellung einer Wachsbasis (1) oder einer Prothesenbasis (1) zur Herstellung einer Dentalprothese, bei dem ein virtuelles CAD-Modell der Dentalprothese zur Herstellung der physischen Dentalprothese oder der physischen Prothesenbasis (1) verwendet wird, wobei aus dem CAD-Modell der Dentalprothese ein virtuelles Modell der Prothesenbasis (1) berechnet wird, **gekennzeichnet durch** die folgenden chronologischen Schritte:
A) Einscannen zumindest eines dreidimensionalen Bereichs eines physischen Modells (4) der Mundraumsituation eines Patienten und Erstellen eines Datensatzes D1 auf Basis dieses Scans oder
Intraoralscan eines dreidimensionalen Bereichs der Mundraumsituation des Patienten und Herstellung eines physischen Modells (4) der Mundraumsituation eines Patienten auf Basis des Intraoralscans und Erstellen eines Datensatzes D1 auf der Basis des Intraoralscans;
B) Aufbringen einer Wachsschicht (12) oder einer Kunststoffschicht (12) auf das physische Modell (4) der Mundraumsituation;
D) Berechnung einer virtuellen Oberfläche als eine in der Wachsschicht (12) oder in der Kunststoffschicht (12) zu erzeugenden Oberfläche, wobei die Berechnung auf Basis des virtuellen Modells der Prothesenbasis (1) und des Datensatzes D1 erfolgt und wobei in der zu erzeugenden Oberfläche Positionierhilfen (2) zur Positionierung von Prothesenzähnen (16) vorgesehen werden; und
E) Herstellen der Wachsbasis (1) aus der Wachsschicht (12) oder der Prothesenbasis (1) aus der Kunststoffschicht (12) mit einem subtraktiven CAM-Verfahren auf Basis der in Schritt D) berechneten virtuellen Oberfläche.

2. Verfahren nach Anspruch 1, **gekennzeichnet durch**
einen Schritt C), der zwischen den Schritten B) und D) erfolgt:
C) Einscannen von zumindest einem Bereich des mit der Wachsschicht (12) oder der Kunststoffschicht (12) belegten physischen Modells (4) der Mundraumsituation und Erstellen eines zweiten Datensatzes D2 auf Basis dieses Scans.

3. Verfahren nach Anspruch 2, **dadurch gekennzeichnet, dass**
in Schritt C) die freie Oberfläche der Wachsschicht (12) oder der Kunststoffschicht (12) auf dem physischen Modell (4) des Mundraums zumindest bereichsweise eingescannt wird und der Scan zu dem zweiten Datensatz D2 verarbeitet wird, wobei bei der Berechnung der virtuellen Oberfläche als die zu erzeugende Oberfläche in der Wachsschicht (12) oder in der Kunststoffschicht (12) auf Basis des CAD-Modells der Prothesenbasis (1), auf Basis des ersten Datensatzes D1 und auf Basis des zweiten Datensatzes D2 erfolgt.

4. Verfahren nach einem der vorangehenden Ansprüche, **dadurch gekennzeichnet, dass**
die Oberfläche, die in der Wachsschicht (12) oder der Kunststoffschicht (12) erzeugt wird, der Auflagefläche der Wachsschicht (12) oder der Kunststoffschicht (12) auf dem physischen Modell (4) des Mundraums gegenüber liegt.

5. Verfahren nach einem der vorangehenden Ansprüche, **dadurch gekennzeichnet, dass**
die Positionierhilfen (2) eine eindeutige Form derart aufweisen, dass die Lage und die Position der Prothesenzähne (16) zueinander eindeutig vorgegeben wird, wenn die Prothesenzähne (16) flächenschlüssig in die Positionierhilfen (2) der Prothesenbasis (1) eingesetzt sind oder wenn die Prothesenzähne (16) flächenschlüssig in die Positionierhilfen (2) der Wachsbasis (1) eingesetzt sind.

6. Verfahren nach einem der vorangehenden Ansprüche, **dadurch gekennzeichnet, dass**
die Herstellung der Wachsbasis (1) aus der Wachsschicht (12) oder die Herstellung der Prothesenbasis (1) aus der Kunststoffschicht (12) mit einem subtraktiven CAM-Verfahren erfolgt, während die Wachsschicht (12) oder die Kunststoffschicht (12) auf dem physischen Modell (4) des Mundraums angeordnet ist.

7. Verfahren nach einem der vorangehenden Ansprüche, **dadurch gekennzeichnet, dass**
beim Einscannen des zumindest einen Bereichs des physischen Modells (4) der Mundraumsituation in Schritt A) zumindest die Oberflächen eingescannt werden, auf die die Wachsschicht (12) oder die Kunststoffschicht (12) anschließend aufgebracht wird oder beim Intraoralscan der Mundraumsituation des Patienten in Schritt A) zumindest die Oberflächen eingescannt werden, auf die die Wachsschicht (12) oder die Kunststoffschicht (12) bei dem physischen Modell (4) aufgebracht wird.

8. Verfahren nach einem der vorangehenden Ansprüche, **dadurch gekennzeichnet, dass**
das Aufbringen der Wachsschicht (12) oder der Kunststoffschicht (12) auf das physische Modell (4) der Mundraumsituation mit Hilfe vorgeformter Wachskörper, insbesondere mit Hilfe von zumindest einer Wachsplatte, oder mit Hilfe weicher vorgeformter Kunststoffkörper, insbesondere mit Hilfe von zumindest einer Kunststoffplatte, durchgeführt wird und/oder ein Abformlöffel zum Aufbringen auf dem physischen Modell (4) des Mundraums verwendet wird.

9. Verfahren nach einem der vorangehenden Ansprüche, **dadurch gekennzeichnet, dass**
das physische Modell (4) des Mundraums vor Schritt A) durch einen Abdruck des Oberkiefers und/oder einen Abdruck des Oberkiefers im Mundraum des Patienten gewonnen wird, wobei vorzugsweise der Abdruck mit Gips oder einem Kunststoff ausgegossen wird, um ein Gips-Modell (4) oder ein Kunststoff-Modell (4) als physisches Modell (4) der Mundraumsituation zu erzeugen.

10. Verfahren nach einem der vorangehenden Ansprüche, **dadurch gekennzeichnet, dass**
das virtuelle CAD-Modell der Dentalprothese und/oder das virtuelle Modell der Prothesenbasis (1) auf Basis des Datensatzes D1 des Scans des physischen Modells (4) der Mundraumsituation oder des Intraoralscans der Mundraumsituation durch ein CAD-Verfahren erzeugt wird.

11. Verfahren nach einem der vorangehenden Ansprüche, **dadurch gekennzeichnet, dass**
zur Umsetzung des subtraktiven CAM-Verfahrens eine computergesteuerte Fräse (14) verwendet wird, bevorzugt eine computergesteuerte 4-Achs- oder 5-Achs-Fräse.

12. Verfahren nach einem der vorangehenden Ansprüche, **dadurch gekennzeichnet, dass**
zur Herstellung der Wachsbasis (1) oder der Prothesenbasis (1) zumindest ein Befestigungsmittel an dem physischen Modell (4) der Mundraumsituation angeordnet wird, insbesondere an den Stellen des physischen Modells (4) der Mundraumsituation angeordnet wird, die Implantaten entsprechen, oder zumindest ein Befestigungsmittel an einer Schiene und/oder einer Verankerungsvorrichtung befestigt wird, die an dem physischen Modell (4) der Mundraumsituation angeordnet wird oder werden, insbesondere an den Stellen des physischen Modells (4) der Mundraumsituation angeordnet wird oder werden, die Implantaten entsprechen, und
anschließend in Schritt B) die Wachsschicht (12) oder die Kunststoffschicht (12) derart auf das physische Modell (4) der Mundraumsituation aufgebracht wird, dass die Wachsschicht (12) oder die Kunststoffschicht (12) mit dem zumindest einen Befestigungsmittel verbunden ist.

13. Verfahren nach Anspruch 12, **dadurch gekennzeichnet, dass**
der Scan des physischen Modells (1) der Mundraumsituation oder der Intraoralscan mit Implantaten, mit zumindest einem eingesetzten Steg und/oder mit zumindest einer Verankerungsvorrichtung am physischen Modell oder im Mundraum durchgeführt wird, besonders bevorzugt mit zumindest einem an den Implantaten, an dem zumindest einen eingesetzten Steg und/oder an der zumindest einen Verankerungsvorrichtung befestigten Befestigungsmittel.

14. Verfahren zur Herstellung einer Dentalprothese oder einer Wachsaufstellung für eine Dentalprothese mit einem Verfahren nach einem der vorangehenden Ansprüche, **dadurch gekennzeichnet, dass**
nach Schritt E) ein Schritt F) erfolgt:
F) Einsetzen von Prothesenzähnen (16) in die Positionierhilfen (2) der Wachsbasis (1), die in Schritt E) in der Oberfläche der Wachsschicht (12) erzeugt wurden, zur Herstellung der Wachsaufstellung, oder Einsetzen und Befestigen, insbesondere Einkleben, von Prothesenzähnen (16) in die Positionierhilfen (2) der Prothesenbasis (1), die in Schritt E) in der Oberfläche der Kunststoffschicht (12) erzeugt wurden, zur Herstellung der Dentalprothese, wobei bevorzugt die Dentalprothese nach dem Einsetzen und Befestigen der Prothesenzähne (16) poliert wird und/oder die Verbindung der Prothesenzähne (16) zur Prothesenbasis (1) ausgehärtet wird.

15. Verfahren zur Herstellung einer Dentalprothese aus einer Wachsaufstellung hergestellt mit einem Verfahren nach Anspruch 14, **dadurch gekennzeichnet, dass**
nach Schritt F) die Wachsaufstellung mit den Prothesenzähnen (16) in eine Form eingegossen wird,
das Wachs der Wachsbasis (1) entfernt wird, insbesondere durch Schmelzen des Wachses und durch Herauslösen des Wachses mit Wasserdampf, wobei die Prothesenzähne (16) in der Form verbleiben und die Lage und Position der Prothesenzähne (16) in der Form unverändert bleiben,
der in der Form entstandene Hohlraum wird mit einem Kunststoff ausgegossen, der die Prothesenbasis (1) der Dentalprothesen bildet,
der Kunststoff wird ausgehärtet, wobei die Prothesenzähne (16) mit dem Kunststoff fest verbunden werden,
die Form wird geöffnet und die Dentalprothese aus der Form entnommen, wobei vorzugsweise die Dentalprothese nach dem Entnehmen aus der Form durch Politur und/oder Entgratung nachbearbeitet wird.

16. Wachsbasis (1) oder Prothesenbasis (1) hergestellt mit einem Verfahren nach einem der Ansprüche 1 bis 13.

17. Dentalprothese hergestellt mit einem Verfahren nach einem der Ansprüche 14 oder 15.

## Claims

1. Method for producing a wax base (1) or a prosthesis base (1) for producing a dental prosthesis, with which a virtual CAD model of the dental prosthesis is used for producing the physical dental prosthesis or the physical prosthesis base (1), wherein, from the CAD model of the dental prosthesis, a virtual model of the prosthesis base (1) is calculated, **characterized by** the following chronological steps:
A) Scanning in of at least one three-dimensional region of a physical model (4) of the oral cavity situation of a patient and preparation of a data record D1 on the basis of this scan, or
intra-oral scan of a three-dimensional region of the oral cavity situation of the patient and production of a physical model (4) of the oral cavity situation of a patient on the basis of the intra-oral scan and preparation of a data record D1 on the basis of the intra-oral scan;
B) application of a wax layer (12) or a plastic layer (12) onto the physical model (4) of the oral cavity situation;
D) calculation of a virtual surface as a surface which is to be formed in the wax layer (12) or in the plastic layer (12), wherein the calculation is made on the basis of the virtual model of the prosthesis base (1) and of the data record D1, and wherein positioning aids (2) are provided in the surface which is to be produced, for the positioning of prosthetic teeth (16); and
E) production of the wax base (1) from the wax layer (12) or of the prosthesis base (1) from the plastic layer (12) with a subtractive CAM method, on the basis of the virtual surface calculated in step D).

2. Method according to claim 1, **characterized by** a step C), which takes place between steps B) and D): C) Scanning in of at least one region of the physical model (4), with the wax layer (12) or the plastic layer (12) applied, of the oral cavity situation and production of a second data record D2 on the basis of this scan.

3. Method according to claim 2, **characterized in that**
in step C), the free surface of the wax layer (12) or the plastic layer (12) on the physical model (4) of the oral cavity is scanned in at least in some regions, and the scan is processed to the second data record D2, wherein the calculation of the virtual surface, as the surface to be produced, takes place in the wax layer (12) or in the plastic layer (12), on the basis of the CAD model of the prosthesis base (1), on the basis of the first data record D1, and on the basis of the second data record D2.

4. Method according to any one of the preceding claims, **characterized in that** the surface which is produced in the wax layer (12) or the plastic layer (12) is located opposite the contact surface of the wax layer (12) or the plastic layer (12) on the physical model (4) of the oral cavity.

5. Method according to any one of the preceding claims, **characterized in that** the positioning aids (2) exhibit a distinctive form, in such a way that the location and the position of the prosthetic teeth (16) in relation to one another is distinctively and unambiguously determined when the prosthetic teeth (16) are inserted with flush surfaces into the positioning aids (2) of the prosthesis base (1) or when the prosthetic teeth (16) are inserted with flush surfaces into the positioning aids (2) of the wax base (1).

6. Method according to any one of the preceding claims, **characterized in that** the producing of the wax base (1) from the wax layer (12) or the producing of the prosthesis base (1) from the plastic layer (12) takes place with a subtractive CAM method, while the wax layer (12) or the plastic layer (12) is arranged on the physical model (4) of the oral cavity.

7. Method according to any one of the preceding claims, **characterized in that**, at the scanning in of the at least one region of the physical model (4) of the oral cavity situation in step A) at least those surfaces are scanned in onto which the wax layer (12) or the plastic layer (12) are subsequently applied, or, at the intra-oral scan of the oral cavity situation of the patient in step A), at least those surfaces are scanned in onto which the wax layer (12) or the plastic layer (12) are applied in respect of the physical model (4).

8. Method according to any one of the preceding claims, **characterized in that** the application of the wax layer (12) or the plastic layer (12) onto the physical model (4) of the oral cavity situation is carried out with the aid of preformed wax bodies, in particular with the aid of at least one wax plate, or with the aid of soft preformed plastic bodies, in particular with the aid of at least one plastic plate, and/or a molding spoon for the application on the physical model (4) of the oral cavity.

9. Method according to any one of the preceding claims, **characterized in that** the physical model (4) of the oral cavity is acquired before step A) by means of an impression of the upper jaw and/or an impression of the upper jaw in the oral cavity of the patient, wherein preferably the impression is cast with gypsum plaster or a plastic, in order to produce a gypsum model (4) or a plastic model (4) as a physical model (4) of the oral cavity situation.

10. Method according to any one of the preceding claims, **characterized in that** the virtual CAD model of the dental prosthesis and/or the virtual model of the prosthesis base (1) is produced on the basis of the data record D1 of the scan of the physical model (4) of the oral cavity situation or of the intra-oral scan of the oral cavity situation, by means of a CAD process.

11. Method according to any one of the preceding claims, **characterized in that**, in order to implement the subtractive CAM process, a computer-controlled milling device (14) is used, preferably a computer-controlled 4-axis or 5-axis milling device.

12. Method according to any one of the preceding claims, **characterized in that**, for producing the wax base (1) or the prosthesis base (1), at least one securing means is arranged at the physical model (4) of the oral cavity situation, in particular at the points of the physical model (4) of the oral cavity situation which correspond to the implants, or at least one securing means is secured to a rail and/or an anchoring device, which is/are arranged at the physical model (4) of the oral cavity situation, in particular at the points of the physical model (4) of the oral cavity situation which correspond to the implants, and,
following this, in step B), the wax layer (12) or the plastic layer (12) is applied onto the physical model (4) of the oral cavity situation in such a way that the wax layer (12) or the plastic layer (12) is connected to the at least one securing means.

13. Method according to claim 12, **characterized in that**
the scan is carried out of the physical model (1) of the oral cavity situation, or the intra-oral scan with implants, with at least one web inserted, and/or with at least one anchoring device at the physical model or in the oral cavity, particularly preferably with at least one securing means secured to the implants, to the at least one inserted web, and/or to the at least one anchoring device.

14. Method for producing a dental prosthesis or a wax set-up for a dental prosthesis, with a method according to any one of the preceding claims, **characterized in that**
after step E), a step F) takes place:
F) Inserting of prosthetic teeth (16) into the positioning aids (2) of the wax base (1), which in step E) were produced in the surface of the wax layer (12), for producing the wax set-up, or inserting and securing, in particular adhesive bonding in place, of prosthetic teeth (16) into the positioning aids (2) of the prosthesis base (1), which in step E) were produced in the surface of the plastic layer (12), for producing the dental prosthesis, wherein, preferably, after the insertion and securing of the prosthetic teeth (16), the dental prosthesis is polished and/or the connection of the prosthetic teeth (16) to the prosthesis base (1) is hardened.

15. Method for producing a dental prosthesis from a wax set-up produced with a method according to claim 14, **characterized in that**
after step F), the wax set-up is cast with the prosthetic teeth (16) into a mold, the wax of the wax base (1) is removed, in particular by melting the wax and by releasing the wax with water vapor or steam, wherein the prosthetic teeth (16) remain in the mold, and the location and position of the prosthetic teeth (16) in the mouth remain unchanged,
the cavity which is created in the mold is cast out with a plastic, which forms the prosthesis base (1) of the dental prostheses,
the plastic is hardened, wherein the prosthetic teeth (16) are securely connected to the plastic,
the mold is opened and the dental prosthesis is removed from the mold, wherein preferably the dental prosthesis is subsequently processed after removal from the mold by polishing and/or deburring.

16. Wax base (1) or prosthesis base (1) produced with a method according to any one of claims 1 to 13.

17. Dental prosthesis produced with a method according to one of claims 14 or 15.

## Revendications

1. Procédé de fabrication d'une base de cire (1) ou d'une base de prothèse (1) destinée à la fabrication d'une prothèse dentaire, dans lequel un modèle par CAO virtuel de la prothèse dentaire est employé pour la fabrication de la prothèse dentaire physique ou de la base de prothèse (1) physique, où un modèle virtuel de la base de prothèse (1) est calculé à partir du modèle par CAO de la prothèse dentaire, **caractérisé par** les étapes chronologiques suivantes :
A) balayage d'au moins une région en trois dimensions d'un modèle (4) physique de l'état de l'espace buccal d'un patient et établissement d'un ensemble de données D1 basé sur ce balayage, ou
balayage intra oral d'une région en trois dimensions de l'état de l'espace buccal du patient et fabrication d'un modèle physique (4) de l'état de l'espace buccal d'un patient basé sur le balayage intra oral et établissement d'un ensemble de données D1 basé sur le balayage intra oral ;
B) application d'une couche de cire (12) ou d'une couche de matière synthétique (12) sur le modèle physique (4) de l'état de l'espace buccal ;
D) calcul d'une surface virtuelle servant de surface de création dans la couche de cire (12) ou dans la couche de matière synthétique (12), où le calcul a lieu sur la base du modèle virtuel de la base de prothèse (1) et de l'ensemble de données D1, et où des aides au positionnement (2) sont prévues dans la surface de création pour le positionnement de dents prothétiques (16) ; et
E) fabrication de la base de cire (1) à partir d'une couche de cire (12) ou de la base de prothèse (1) à partir de la couche de matière synthétique (12) avec un procédé de FAO soustractif basé sur la surface virtuelle calculée dans l'étape D).

2. Procédé selon la revendication 1, **caractérisé par**
une étape C), qui a lieu entre l'étape B) et l'étape D) :
C) de balayage d'au moins une région du modèle physique (4) de l'état de l'espace buccal revêtu avec la couche de cire (12) ou avec la couche de matière synthétique (12) et d'établissement d'un deuxième ensemble de données D2 basé sur ce balayage.

3. Procédé selon la revendication 2, **caractérisé en ce que**
dans l'étape C), la surface libre de la couche de cire (12) ou de la couche de matière synthétique (12) est balayée au moins par endroits par rapport au modèle physique (4) de l'espace buccal et le balayage est exploité pour donner le deuxième ensemble de données D2, où, lors du calcul de la surface virtuelle servant de surface de création dans la couche de cire (12) ou dans la couche de matière synthétique (12), le calcul a lieu sur la base du modèle par CAO de la base de prothèse (1), sur la base du premier ensemble de données D1 et sur la base du deuxième ensemble de données D2.

4. Procédé selon l'une des revendications précédentes, **caractérisé en ce que** la surface qui est créée dans la couche de cire (12) ou dans la couche de matière synthétique (12) est située vis-à-vis de la surface d'appui de la couche de cire (12) ou de la couche en matière synthétique (12) sur le modèle physique (4) de l'espace buccal.

5. Procédé selon l'une des revendications précédentes, **caractérisé en ce que** les aides au positionnement (2) présentent une forme explicite de telle manière que l'orientation et la position des dents prothétiques (16) les unes par rapport aux autres est fixée de manière claire, lorsque les dents prothétiques (16) sont insérées de manière à être jointives par leurs surfaces dans les aides au positionnement (2) de la base de prothèse (1) ou lorsque les dents prothétiques (16) sont insérées de manière à être jointives par leurs surfaces dans les aides au positionnement (2) de la base de cire (1).

6. Procédé selon l'une des revendications précédentes, **caractérisé en ce que** la fabrication de la base de cire (1) à partir de la couche de cire (12) ou la fabrication de la base de prothèse (1) à partir de la couche de matière synthétique (12) a lieu par un procédé de FAO soustractif, tandis que la couche de cire (12) ou la couche de matière synthétique (12) est posée sur le modèle physique (4) de l'espace buccal.

7. Procédé selon l'une des revendications précédentes, **caractérisé en ce que** lors du balayage de l'au moins une région du modèle physique (4) de l'état de l'espace buccal dans l'étape A), au moins les surfaces sur lesquelles est ensuite rapportée la couche de cire (12), ou la couche de matière synthétique (12), sont balayées ou lors du balayage intra oral de l'état de l'espace buccal du patient dans l'étape A), au moins les surfaces sur lesquelles la couche de cire (12), ou la couche de matière synthétique (12), est rapportée, sont balayées dans le cas du modèle physique (4).

8. Procédé selon l'une des revendications précédentes, **caractérisé en ce que** l'application de la couche de cire (12), ou de la couche de matière synthétique (12), sur le modèle physique (4) de l'état de l'espace buccal est effectuée à l'aide de corps en cire préformés, notamment à l'aide d'au moins une plaque de cire, ou à l'aide d'organe plastiques préformés, notamment à l'aide d'au moins une plaque de matière plastique, et/ou une cuiller de prélèvement est employée pour l'application sur le modèle physique (4) de l'espace buccal.

9. Procédé selon l'une des revendications précédentes, **caractérisé en ce que** le modèle physique (4) de l'espace buccal est obtenu avant l'étape A) par une empreinte du maxillaire supérieur et/ou une empreinte du maxillaire supérieur dans l'espace buccal du patient, où, de préférence, l'empreinte est coulée avec du plâtre ou une matière synthétique, afin de créer un modèle en plâtre (4) ou un modèle en matière synthétique (4) servant de modèle physique (4) de l'état de l'espace buccal.

10. Procédé selon l'une des revendications précédentes, **caractérisé en ce que** le modèle par CAO virtuel de la prothèse dentaire et/ou le modèle virtuel de la base de prothèse (1) est créé sur la base de l'ensemble de données D1 du balayage du modèle physique (4) de l'état de l'espace buccal ou du balayage intra oral de l'état de l'espace buccal par un procédé par CAO.

11. Procédé selon l'une des revendications précédentes, **caractérisé en ce que** pour la transposition du procédé de FAO soustractif, on utilise une fraise (14) commandée par ordinateur, de préférence une fraise à 4 axes ou à 5 axes commandée par ordinateur.

12. Procédé selon l'une des revendications précédentes, **caractérisé en ce que**, pour la fabrication de la base de cire (1) ou de la base de prothèse (1), au moins un moyen de fixation est disposé sur le modèle physique (4) de l'état de l'espace buccal, notamment à des endroits du modèle physique (4) de l'espace buccal qui correspondent à des implants, ou au moins un moyen de fixation est fixé sur un rail et/ou un dispositif d'ancrage qui est ou sera disposé sur le modèle physique (4) de l'état de l'espace buccal, notamment à des endroits du modèle physique (4) de l'état de l'espace buccal qui correspondent à des implants, et
ensuite, dans l'étape B), la couche de cire (12), ou la couche de matière synthétique (12), est rapportée sur le modèle physique (4) de l'état de l'espace buccal de sorte que la couche de cire (12) ou la couche de matière synthétique (12) est reliée avec l'au moins un moyen de fixation.

13. Procédé selon la revendication 12, **caractérisé en ce que**
le balayage du modèle physique (1) de l'état de l'espace buccal ou le balayage intra oral est effectué avec des implants, avec au moins une tige insérée, et/ou avec au moins un dispositif d'ancrage sur le modèle physique ou dans l'espace buccal, de manière particulièrement préférée, avec au moins un moyen de fixation fixé sur l'implant, sur l'au moins une tige insérée, et/ou sur l'au moins un dispositif d'ancrage.

14. Procédé de fabrication d'une prothèse dentaire ou d'une structure en cire pour une prothèse dentaire avec un procédé selon l'une des revendications précédentes, **caractérisé en ce qu'**après
l'étape E), il y a une étape F) :
F) de mise en place de dents prothétiques (16) dans les aides au positionnement (2) de la base de cire (1) qui ont été créées dans la surface de la couche de cire dans l'étape E), pour la fabrication de la structure en cire, ou de mise en place et de fixation, notamment de collage, de dents prothétiques (16) dans les aides au positionnement (2) de la base de prothèse (1), qui ont été créées dans la surface de la couche de matière synthétique (12) dans l'étape E), pour la fabrication de la prothèse dentaire, où, de préférence, la prothèse dentaire est polie après la mise en place et la fixation des dents prothétiques (16), et/ou la liaison des dents prothétiques (16) vers la base de prothèse (1) est durcie.

15. Procédé de fabrication d'une prothèse dentaire à partir d'une structure en cire fabriquée avec un procédé selon la revendication 14, **caractérisé en ce qu'**après l'étape F), la structure en cire avec les dents prothétiques (16) est coulée dans un moule,
**que** la cire de la base de cire (1) est enlevée, en particulier, par la fusion de la cire et par le détachement de la cire avec de la vapeur d'eau, où les dents prothétiques (16) restent dans le moule et l'orientation et la position des dents prothétiques (16) restent inchangées dans le moule,
**que** l'espace creux formé dans le moule est comblé avec une matière synthétique qui forme la base de prothèse (1) des prothèses dentaires,
**que** la matière synthétique est durcie, où les dents prothétiques (16) restent solidement reliées avec la matière synthétique,
**que** le moule est ouvert et la prothèse dentaire est prélevée du moule, où, de préférence, la prothèse dentaire est façonnée encore par un polissage et/ou un ébavurage après le prélèvement du moule.

16. Base de cire (1) ou base de prothèse (1) fabriquée avec un procédé selon l'une des revendications 1 à 13.

17. Prothèse dentaire fabriquée avec un procédé selon l'une des revendications 14 ou 15.
